(19) Europäisches Patentamt
European Patent Office
Office européen des brevets

(11) EP 2 213 308 A1

(12) EUROPEAN PATENT APPLICATION

(43) Date of publication:
**04.08.2010 Bulletin 2010/31**

(21) Application number: **10002999.0**

(22) Date of filing: **12.08.2002**

(51) Int Cl.:
***A61K 47/36*** *(2006.01)* ***A61K 9/08*** *(2006.01)*
***A61K 47/10*** *(2006.01)* ***A61P 17/00*** *(2006.01)*
***A61P 27/04*** *(2006.01)* ***C08B 37/12*** *(2006.01)*
***C08J 3/03*** *(2006.01)* ***A23L 1/0532*** *(2006.01)*
***A61K 9/06*** *(2006.01)* ***A23L 1/0526*** *(2006.01)*

(84) Designated Contracting States:
**AT BE BG CH CY CZ DE DK EE ES FI FR GB GR IE IT LI LU MC NL PT SE SK TR**

(30) Priority: **10.08.2001 JP 2001243827**

(62) Document number(s) of the earlier application(s) in accordance with Art. 76 EPC:
**02758837.5 / 1 424 081**

(71) Applicants:
- **TORAY INDUSTRIES INC.**
  **Chuo-ku**
  **Tokyo 103-8666 (JP)**
- **SANTEN PHARMACEUTICAL CO., LTD.**
  **Higashiyodogawa-ku,**
  **Osaka-shi,**
  **Osaka 533-8651 (JP)**

(72) Inventors:
- **Inohara, Masahiro**
  **Nagoya-shi, Aichi 467-0042 (JP)**
- **Yoshikawa, Masahito**
  **Midori-ku,**
  **Nagoya-shi, Aichi 458-0033 (JP)**
- **Taniguchi, Takashi**
  **Yasu-gun, Shiga 520-2324 (JP)**
- **Yokota, Mitsuru**
  **Otsu-shi, Shiga 520-0052 (JP)**
- **Shimoyama, Naoki**
  **Otsu-shi, Shiga 520-0845 (JP)**
- **Ue, Masaki**
  **Kyoto-shi, Kyoto 607-8088 (JP)**
- **Araki, Miho**
  **Otsu-shi, Shiga 520-2141 (JP)**
- **Sugihara, Yukiko**
  **Ikoma-shi, Nara 630-0101 (JP)**
- **Horibe, Yoshihide**
  **Ikoma-shi, Nara 630-0101 (JP)**
- **Kuwano, Mitsuaki**
  **Ikoma-shi, Nara 630-0101 (JP)**

(74) Representative: **Peaucelle, Chantal et al**
**Cabinet Armengaud Aîné**
**3, Avenue Bugeaud**
**75116 Paris (FR)**

Remarks:
This application was filed on 22-03-2010 as a divisional application to the application mentioned under INID code 62.

# (54) Eyedrop comprising agar

(57) It is intended to prepare a composition which contains polysaccharide at a high concentration and yet remains in the state of a liquid having low viscosity to thereby provide drugs, eyedrops, foods, cosmetics, toiletry products having a novel texture or function. The composition in the state of a liquid having low viscosity is obtained by heating polysaccharide at a high concentration in a water-containing liquid and then cooling under applying a shear force, which enables the provision of the above-described drugs. The composition is usable as an aqueous drug vehicle which is free from gelling due to temperature changes during storage and easily applied without pouring and/or streaming down. Eyedrops containing agar have an effect of enhancing ocular drug penetration. Eyedrops containing particulate agar maintain a low viscosity and, achieve easy instillation and impart a favorable feel in instillation.

EP 2 213 308 A1

## Description

### Technical Field

The present invention relates to a polysaccharide-containing composition characterized by containing polysaccharide at a high concentration and yet having a low viscosity. The invention also relates to a composition which enables to prolong a retention time of drugs, pigments, paints, and the like to be contained owing to the use of the composition. Further, the invention relates to an eyedrop which is enhanced ocular drug penetration owing to incorporating agar. Particularly, in the case of using particulate agar as a vehicle, for eyedrops, the obtained eyedrop has excellent properties.

### Background Art

Various gelling agents and thickeners are used in the fields of drugs, foods, cosmetics, toiletry products, and the like, and various products in the state of a gel and a sol are available on the market. Natural polysaccharides and proteins are used in such fields because of their safety and good image, but diversity in texture of the sol and the gel and stability or a reversible control of a gelling temperature and sol/gel states are in demand.

JP-T-2002-514395, JP-A-2000-239147, and the like disclose an achievement of a fluidity by preparing a hydrogel of polysaccharide and then pulverizing the hydrogel under applying a shear force. However, the obtained compositions are high in viscosity and lack in fluid uniformity.

European Patent No. 432835, for example, which is another publication of the polysaccharide-containing composition having low viscosity and fluidity, discloses a fluid composition containing a microgel of polysaccharide and a preparation process thereof, wherein polysaccharide is heated to a temperature above the gelling temperature and then cooled to a temperature below the gelling temperature with performing a shearing process.

Further, the polysaccharide-containing composition having low viscosity and fluidity prepared by a similar process is also disclosed in Japanese Patent No. 2513506, JP-A-2000-119116, and so on. The viscosity of each of the above fluid compositions obtained by the above preparation methods is lowered with the increase in shear force (shear speed) as reviewed in International Journal of Biological Macromolecules, 26 (1999), P255-261*, Fig. 8.*

In addition, many aqueous pharmaceuticals which are in the state of a liquid at a room temperature or below and in the state of a semi-solid or a gel at a body temperature of a mammal to be treated have been disclosed as aqueous pharmaceutical compositions for efficiently releasing a pharmaceutical ingredient in the mammal. However, a problem is the gelling of the aqueous compositions due to a temperature rise during storage.

For example, a heat-gelled aqueous pharmaceutical using pluronic (trade name: PLURONIC) is disclosed in United States Patent No. 4,188,373, and a drug releasing system using a heat-gelled aqueous pharmaceutical is disclosed in United States Patent No. 4,478,822.

A skin cream, for example, used for sun protection or the like is available in the state of a lotion (liquid), a gel, and an ointment, and the lotion-like skin cream is applied most easily though it entails a disadvantage of streaming down.

Since agar contains dietary fiber, it is expected to have an effect of improving bowel movement. Agar is gelled at a concentration of 0.1 wt.% or more, however, a development thereof as a drinkable food which is easily ingested has been desired.

As a therapy for eye diseases, instillation of drug is generally performed. It is needless to say that a therapeutic effect of the instillation on eye diseases depends on an efficacy of the drug itself, and, in order to fully exhibit the drug efficacy, how to deliver drugs efficiently in eyes is considered important.

Various studies have been made in order to enhance ocular drug penetration, and, in a known technology of enhancing ocular drug penetration (JP-B-60-56684), for example, a viscosity of an eyedrop is increased by using carboxylvinyl polymer (CVP) as a vehicle, to increase a retention time of the drug on the eye surface and to achieve a sustained release effect. This technology exploits the characteristic of CVP, i.e., the property of expeditiously increasing the viscosity of an eyedrop with a least amount.

The above technology is favorably used for eye ointments. However, since eyedrops need to be instilled in the state of droplets, development of eyedrops which are instilled in the state of droplets and attain excellent ocular drug penetration has been desired. For example, a study of CVP exhibiting a Newton viscosity and capable of instillation in the state of droplets (Japanese Patent No. 2873530), a study of polymers which are instilled in the state of droplets and gelled rapidly at a contact with tear fluid after instillation (Japanese Patent No. 2814637), polysaccharide capable of liquid-gel phase transition (JP-B-6-67853), and so forth have been reported.

Polysaccharides in general, of which agar is representative, are gelled when a temperature is lowered from a temperature over a gel transition temperature which is equal to or higher than a room temperature in the case where a content thereof is 0.1 wt.% or more and, therefore, cannot be used as a liquid composition having fluidity at the room temperature. Accordingly, an object of the present invention is to prepare a composition which contains polysaccharide, of which agar is representative, at a high concentration and yet remains in the state of a liquid having a low viscosity at

a room temperature thereby to provide drugs, eyedrops, foods, cosmetics, toiletry products, etc. having a novel texture and/or function. A further object of the present invention is to provide the bowel movement improving foods in the state of liquid, which are more easily taken, not in the state of jelly-like foods, when the composition is used for foods having the effect of improving bowel movement.

In terms of ophthalmic pharmaceuticals, since it has been difficult to prepare a satisfactory vehicle for eyedrops by the conventional techniques, there has been a demand for development of a novel vehicle which enhances ocular drug penetration and can be administered in the state of droplets.

Important subjects in the development of the novel vehicle are:

1) no problem in safety;
2) easy availability of raw materials;
3) easy processing and handling;
4) easy instillation in the state of droplets;
5) excellent texture (imparting favorable feel in instillation); and
6) excellent ocular drug penetration.

Disclosure of the Invention

The present inventors have conducted extensive studies in order to solve the above problem and have found that it is possible to obtain an aqueous composition containing polysaccharide, of which agar is representative, at a concentration of 0.1 wt. % or more and yet remaining in the state of a liquid having a low viscosity by heating the polysaccharide such as agar in a water-containing liquid and then cooling under applying a stress such as a shear force, thereby accomplishing the present invention.

More specifically, a first invention relates to a polysaccharide-containing composition characterized by containing from 0.1 to 30 wt.% of polysaccharide and having a viscosity of 700 mPa•s or less, the viscosity being measured by using a B type viscometer (rotor No.2) under the conditions of 20˚C and 60 rpm, and a process for preparing the above-mentioned composition characterized by heating polysaccharide in a water-containing liquid and cooling under applying a shear force. As a result of further extensive studies, the invention relates to the polysaccharide-containing composition and the process for preparation thereof according to claim 1, **characterized in that** the viscosity of the polysaccharide-containing composition is increased with an increase in the shear force (shear speed). With the use of the polysaccharide-containing composition and the thickening composition, it is possible to provide novel drugs, ocular penetration enhancers, foods and cosmetics.

The inventors have also focused on agar which is used widely (for foods, for example) and have conducted extensive studies in order to solve the above problem detected with the use thereof in the field of ophthalmology thereby to find that it is possible to prepare a composition containing particulate agar at a high concentration and being in the state of a liquid having a desired viscosity by heating and dissolving agar in an aqueous solution and then cooling the mixture under applying a stress. In general, gelling occurs at a room temperature when an agar content is 0.1 wt.% or more. However, this particulate agar is **characterized in that** it maintains the liquidity though containing agar at a high concentration and has a relatively low viscosity. Then, the inventors have found that it is possible to achieve an excellent ocular drug penetration through the use of the particulate agar as a vehicle of eyedrops and, further, to provide novel eyedrops and ocular penetration enhancers.

Accordingly, a second invention relates to an eyedrop enhanced ocular drug penetration by incorporating agar. Particularly, an eyedrop having superior characteristics is obtained by the use of the particulate agar as of vehicle. Further, the invention relates to an ocular drug penetration enhancer characterized by using agar as a vehicle.

The first invention relates to an agar-containing composition characterized by containing from 0.1 to 30 wt.% of polysaccharide and having a viscosity of 700 mPa•s or less, the viscosity being measured by using a B type viscometer (rotor No.2) under the conditions of 20˚C and 60 rpm, and the preparation process thereof. Conventionally, agar has been used as food in the state of a gel, and its property has been indicated by a gel strength (index for indicating a hardness of a gel) which is an evaluation as a solid. The gist of the invention is that agar can be obtained in the state of a liquid composition, not in the state of a gel, by heating agar in a water-containing liquid and then cooling under applying a shear force. The invention will hereinafter be described in detail.

Polysaccharides usable in this invention are, in the broadest sense, carbohydrates each of which generates monosaccharide having two or more molecules through hydrolysis, such as disaccharide, trisaccharide, tetrasaccharide and like oligosaccharides, and examples thereof are natural polysaccharides, those obtained by processing the natural polysaccharides, synthetic polysaccharides, and so forth. Among the above, polysaccharides derived from plants, particularly from seaweeds, may preferably be used. Examples of the polysaccharides obtained from plants may be typical ones in any forms such as those described in Basics of Glycochemistry, written by Kimiko Abu and Nobuko Seno, Published by Kodansha in 1984, and a plurality of the polysaccharides may be used in combination. Specific examples

thereof may be agar, agarose, agaropectin, starch, amylose, amylopectin, isolichenan, laminaran, lichanan, glucan, inulin, levan, fructan, galactan, manna, xylan, arabinan, pentozan, alginic acid, pectinic acid, protuberic acid, chitin, colominic acid, porphyran, fucoidan, ascophyllan, carrageenan, pectin, locust bean gum, guar gum, tamarind gum, tara gum, arabic gum, gellan gum, and the like. Among the above polysaccharides, those obtained from seaweeds such as agar, agarose, agaropectin, laminaran, fructan, galactan, pentozan, alginic acid, chitin, porphyran, fucoidan, ascophyllan, carrageenan, and the like may preferably be used. and, more preferably, agar, agarose, and agaropectin may be used.

Agar is a sort of polysaccharide contained in the cell wall matrix of various red algae such as Gelidiales and Gracilarlaceae, which is obtained by extraction using hot water. Agar is highly safe as is apparent from its conventional use for foods and its insertion in Japanese Pharmacopoeia and actually used widely for foods and the like. Agar is not a uniform substance and typically consists of agarose which does not contain any sulfuric acid group and agaropectin which contains the sulfuric acid group and the like. A proportion of agarose varies depending on the type of the red alga, and the proportion of agarose in Gelidiales is about 70%.

Agarose is a sort of polysaccharide having a straight chain structure of repetitive bonding wherein D-galactose alternates with 3,6-anhydro-L-galactose residue by β-(1→4) bonding and α-(1→3) bonding as shown in Chemical Formula 1, and agaropectin is a mixture of acidic polysaccharides which has the framework of agarose and contains a sulfuric acid group (Chem. 2), a methoxyl group (Chem. 3), and a pyruvic acid residue (Chem. 4), and D-glucuronic acid (Chem. 5) in various proportions.

1

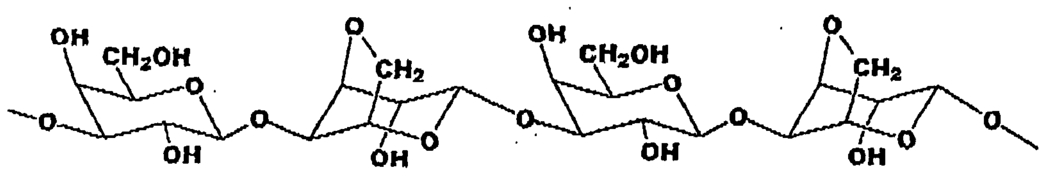

2

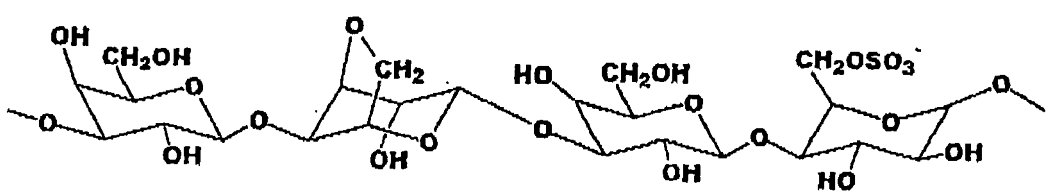

3

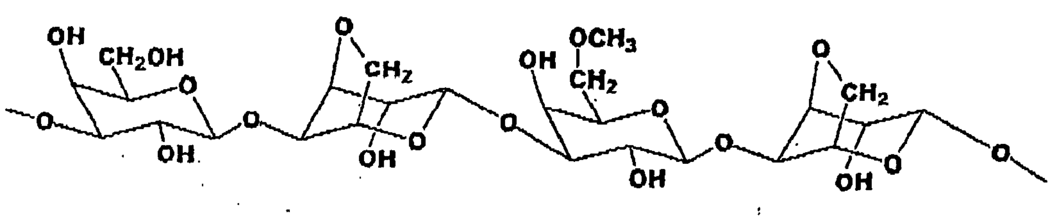

4

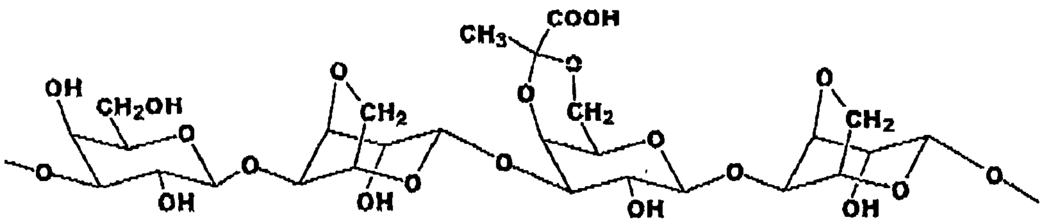

5

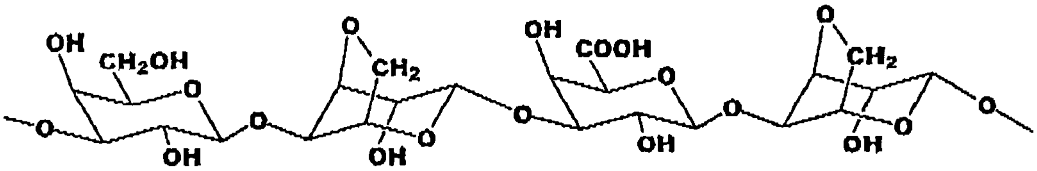

In the case of using agar, the production process is not limited, but agar produced by an industrial process may preferably be used from the viewpoint of stable supply. A weight-average molecular weight of agar to be used may preferably be from 5,000 to 1,200,000, more preferably from 30,000 to 800,000, further preferably from 50,000 to 500,000. By using agar whose weight-average molecular weight is in the above range, agar having a good fluidity and superior handling easiness is obtained. Particularly, when such agar is used as a vehicle or base for drugs, for example, the drugs can be provided in various forms such as instillation and spray, and, thanks to a retention property of the drugs, which is one of objects of this invention, more excellent drugs are obtained.

The polysaccharide-containing composition of the invention can be a mixture of polysaccharide and a solvent such as water; a mixture of polysaccharide, a solvent such as water, and an ingredient other than the solvent; and the like.

A concentration of polysaccharide to be employed in this invention may be from 0.1 to 30 wt.%. In the case where the composition is used for foods, an effect of improving bowel movement of the composition is increased with an increase in agar concentration. When polysaccharide is used as a vehicle or base for something other than foods, a retention property of function agents such as drugs is achieved, and such effect is increased with the increase in content of polysaccharide. A lower limit of the polysaccharide content may preferably be 0.2 wt. % or more, more preferably 0.3 wt.% or more, and further preferably 0.5 wt.% or more. Though an upper limit thereof is not particularly limited so far as the content does not deteriorate handling easiness of an end product, it may preferably be 30 wt.% or less, more preferably 10 wt.% or less, further preferably 5 wt. % or less, and most preferably 1. 5 wt.% or less. In the case of using agar as polysaccharide, gelling occurs when a concentration is too high, thereby causing a viscosity measured by using a B type viscometer under the conditions of 20˚C and 60 rpm to undesirably exceed 700 mPa•s depending on the type of agar to be used. Therefore, the agar concentration may preferably be 5 wt.% or less.

Examples of agar to be preferably used in the invention are UP-6, UP-16, UP-37, M-7, M-9, AX-30, AX-100, AX-200, BX-30, HX-100, BX-200, PS-5, PS-6, PS-7, and PS-8, all of which are manufactured by Ina Food Industry Co., Ltd., and the like. The above agars can be used alone or in combination of two or more.

A viscosity of the polysaccharide-containing composition of the invention measured by a B type viscometer under the conditions of 20˚C and 60 rpm may be 700 mPa•s or less, preferably 500 mPa•s or less, more preferably 150 mPa•s or less, and the most preferably 100 mPa•s or less. Though a lower limit of the viscosity is not particularly limited, the viscosity may be about 1 mPa•s from the practical point of view.

Polysaccharides in general (agar, for example) are used in a content of 0.1 wt.% or more and are gelled when heated in an aqueous medium and then cooled to a room temperature to naturally exhibit a remarkably high viscosity as is apparent from the heretofore exploitation of its gel characteristics. Partioularly, polysaccharides are gelled when the content thereof is 0.1 wt.% or more in most cases, and they are completely gelled when the content is 0.3 wt.% or more. Thus, it has been impossible to obtain a polysaccharide-containing composition having the viscosity of 700 mPa•s or less. The polysaccharide-containing composition of the invention has the above-described special characteristics, and the composition is obtained by a process described later in this specification, for example.

An optimum range of viscosity from the stand point of application is such that a viscosity during storage may

preferably be 200 mPa•s or less, more preferably 150 mPa•s or less, and further preferably 100 mPa•s or less, though it depends on the type of the application (the above values of viscosity are measured by the B type viscometer under the conditions of 20˚C and the number of revolutions of 60 rpm). In the case where the composition is used for an ointment, the composition having a viscosity of 700 mPa•s or more are usable, though such viscosity is not preferred due to its inferiority in hygiene.

The polysaccharide-containing composition of the invention may preferably contain an aqueous medium as one of the ingredients. The aqueous medium is a substance in the state of a liquid mainly containing water. Though other ingredients of the medium than water are not particularly limited, a water content may preferably exceed 80 wt.%, more preferably 90 wt.%.

The aqueous medium may preferably contain a water-soluble compound. The water-soluble compound is not particularly limited so far as it dissolves into water to give a stable composition. Examples of the water-soluble compound are alcohols such as methanol, ethanol, ethyleneglycol, propyleneglycol, and glycerin, various surfactants, emulsifiers, dispersants, isotonic agents, and the like. In addition to the above low molecule compounds, water-soluble high polymer compounds such as polyethyleneglycol and polyvinylalcohol may be used. The above water-soluble compounds may be used alone or in combination of two or more.

A part or a whole of polysaccharide present in the polysaccharide-containing composition of the invention may be, in some cases, in the state of a partioulate gel. Preferably, a part of polysaccharide is in the state of the particulate gel. Further, the particulate gel may preferably be dispersed uniformly.

In the case where the particulate gel is formed, the shape of the gel may be spherical, elliptical, or amorphous, though it is not particularly limited. The shape may preferably be spherical because it does not cause foreign-body sensation, and a diameter of the particulate gel may preferably be 100 $\mu$m or less, more preferably 50 $\mu$m or less, further preferably 20 $\mu$m, and most preferably 10 $\mu$m or less. When the diameter is too large, a storage stability of the composition can be adversely affected and, when such composition is used for eyedrops, for example, the eyedrop can entail a functional disadvantage such as the foreign-body sensation after instillation.

Next, the process for preparing the polysaccharide-containing composition of the invention will be described using specific examples.

First, a predetermined amount of polysaccharide, a predetermined amount of an aqueous medium, and other ingredient(s) as required are mixed, and then the resulting mixture is heated to dissolve polysaccharide. Any of conventional methods can be employed as heating means. The mixture is heated to a gel transition temperature or above, preferably to a temperature higher than the gel transition temperature by 20˚C or more. The mixture may sometimes need to be boiled. Preferably, the obtained liquid is transparent and uniform. Then, the mixture is cooled under applying a stress.

A method of applying the stress may be vibration, stirring, compression, pulverization, and the like. Because it is necessary to apply a shear force to the liquid, the stirring is the most preferable. More specifically, a stirring appliance such as a magnetic stirrer, a mechanical stirrer, a mixer, a shaker, a rotor, and a homogenizer may be used or a human power stirring may be performed.

Cooling means may be air-cooling, water-cooling, ice-cooling, solvent-cooling, wind-cooling, or the like, and any known means may be employed. A cooling speed may be selected depending on characteristics of the polysaccharide to be used or characteristics of the polysaccharide-containing composition to be obtained, but, in general, the air-cooling, the water-cooling, or the ice-cooling is performed. In the case of rapidly cooling the mixture by the water-cooling or the ice-cooling, it is necessary to increase a stirring force so as to prevent gelling. Cooling to the gel transition temperature or below is sufficient in principle; however, from the practical point of view, the mixture is cooled to a temperature lower than the gel transition temperature by 20˚C or more or to about 20˚C since the polysaccharide-containing composition of the invention is usually used at a room temperature or below. It is preferred to continue the stirring for 10 minutes or more after the temperature of the composition reaches the aiming temperature in order to prevent the gelling.

In the stirring, a viscosity is increased with a lowering in temperature of the polysaccharide-containing composition, and it is necessary to stir the composition with resistance against the viscosity. The stirring means may preferably be a powerful one. More specifically, it is preferred to stir the composition in such a manner that the Reynolds number of the stirring is 100 or more.

In the case of using a stirrer having a relatively small shear force, such as the magnetic stirrer or the mechanical stirrer, as the stirring means, a viscosity of the composition to be obtained is reduced with the increase in the shear force (shear speed) as is reported in the aforementioned literature Journal of Biologlcal Macromolecules, 26 (1999), p. 255-261*, Fig. 8*. In the case of applying a larger shear force by using stress applying means having a large shear force (shear speed) such as the homogenizer (for example, T. K. HOMO MIXER manufactured by Tokushu Kika Kogyo Co., Ltd.), it is preferred to shear the composition with a shear force belonging to a range of shear force which contributes to an increase in viscosity of the product as described in embodiments in datail later in this specification. By employing such method, it is possible to reduce the number or the size of gel particles, thereby enabling to achieve an excellent feel in instillation when the composition is used as an ingredient of eyedrops or, when the composition is used in

combination with a function agent, enabling the function agent to exhibit its effect more efficiently.

In this invention, the polysaccharide-containing composition is prepared so as to achieve a viscosity of 700 mPa•s or less, measured by using the B type viscometer (rotor No. 2) under the conditions of 20˚C and 60 rpm. The viscosity is achieved by combining the stress applying means, the stress applying conditions, and the like depending on the type and the concentration of polysaccharide to be used. Examples of the combinations are described in the following embodiments.

It is possible to prepare the agar-containing composition having a low viscosity and containing agar a high concentration by the above-described process, and the principle thereof can be explained as follows though it has not been clarified. It is considered that the gelling of agar occurs in such a manner that a hydrogen bonding is formed between molecular chains of agar molecules so that the agar molecules incorporate water molecules to establish a helical structure, thereby achieving a higher-order and stronger structure. The agar molecules take a random coil molecular structure when a uniform state is achieved by high temperature heating. Then the agar molecules start to establish the helical structure as they are cooled down. However, the agar molecules are prevented from forming the helical structure by applying a strong shear force to them during the cooling, thereby giving a liquid composition having a low viscosity without gelling.

As used herein, the isotonic agent to be preferably used in this invention means a solute contained in an ordinary isotonic solution. The isotonic solution is a solution obtained by adding an isotonic agent to one of two solutions having different osmotic pressures so as to equalize the osmotic pressure of one of the solutions with that of the other solution. The isotonic agent may be used in the case of using the compositions of the invention alone or in combination. An amount of the isotonic agent to be added is not particularly limited so far as it is used for achieving a desired osmotic pressure. Examples of the isotonic agent are glycerin. propyleneglycol, sorbitol, mannitol, sodium chloride, sodium phosphate, boric acid, borax, and the like.

The composition of the invention contains at least polysaccharide and the aqueous compound and has a preferred viscosity property with a viscosity ratio represented by the following equation, thereby exhibiting a desired function when used for a drug or in combination with a function agent. The viscosity ratio X is defined by the following equation:

$$X = Z/Y.$$

In the equation, Y represents a viscosity of the polysaccharide-containing composition containing the aqueous compound measured by using a B type viscometer (rotor No. 2) under the conditions of 20˚C and 60 rpm, and Z represents a viscosity measured by using the B type viscometer (rotor No. 2) under the concutions of 20˚C and 60 rpm after adding 0.9 wt.% NaCl to the polysaccharide-containing composition containing the isotonic agent and the aqueous compound.

If the viscosity ratio X is 1.005 or more, the composition of the invention exhibits characteristics such as hardly streaming down and remaining on an applied portion for a long time owing to the viscosity when contacting with, for example, a physiological fluid containing salt such as sweat and tear fluid. In order to further exhibit the characteristics, the viscosity ratio X may preferably be 1.008 or more, more preferably 1.010 or more. However, when the viscosity ratio X exceeds 3.000, an increase in the viscosity after application may be too rapid, thereby in some cases causing unpleasant feel in the application to cosmetics.

The composition of the invention may be used for drugs for human or animals; vehicles or bases for drugs; foods; cosmetics; toiletry products; and so forth without limitation thereto. More specifically, the composition may be used for food of which a taste remains in the mouth, a sunscreen cream which is hardly taken off with seawater, a cosmetic which is hardly taken off with sweat, a pharmaceutical ointment which is hardly taken off with sweat, an eyedrop capable of suppressing outflow of an pharmaceutical ingredient due to tear fluid, and so forth.

For example, in the case of using the composition of the invention as an ingredient of eardrops, an active ingredient of the eardrops can be either water-soluble or water-insoluble. When using the water-insoluble active ingredient, water-soluble compounds such as ethanol, isopropanol, propyleneglycol, and glycerin; a surfactant; and the like may be properly used as other ingredients.

Examples of the active ingredient (drug) are glutethimide, chloral hydrate, nitrazepam, amobarbital, phenobarbital, and like hypnotic-sedative agents; aspirin, acetaminophen, ibuprofen, flurbiprofen, indomethacin, ketoprofen, diclofenac sodium, tiaramide hydrochloride, piroxicam, flufenamic acid, mefenamic acid, pentazocine and like antipyretic-analgesic-antiphlogistic agents; methyl aminobenzoate, lidocaine, and like local anesthetics; naphazoline nitrate, tetryzoline nitrate, oxymethazoline hydrochloride, tramazoline hydrochloride, and like local angiotonics; chlorpheniramine maleate, sodium cromoglycate, oxatomide, azelastine hydrochloride, ketotifen fumarate, traxanox sodium, amlexanox, and like antiallergic agents; benzethonium chloride and like bactericides; dopamine hydrochloride, ubidecarenone, and like carditonics; propranolol hydrochloride, pindolol, phenytoin, disopyramide, and like antiarrhythmic agents; isosorbide

nitrate, nifedipine, diltiazem hydrochloride, dipyridamole, and like coronary vasodilators; domperidone and like agents for digestive organs; triamcinolone acetonide, dexamethasone, betamethasone sodium phosphate, prednisolone acetate, fluocinonide, beclometasone dipropionate, flunisolide, and like adrenocortical hormones; tranexamic acid and like antiplasminic agents; clotrimazole, miconazole nitrate, ketoconazole, and like antifungal agents; tegafur, fluorouracil, mercaptopurine, and like anti-malignant tumor agents; amoxicillin, ampicillin, caphalexin, cephalotin sodium, ceftizoxime sodium, erythromycin, oxytetracycline hydrochloride, and like antibiotics; insulin, calcitonin salmon, calcitonin chicken, elcatonin, and like calcitonins; urokinaze, TPA, interferon, and like biologically active peptides; influenza vaccine, pig Bordetella infection prophylactic vaccine, hepatitis B vaccine, and like vaccines; and the like. A content of the active ingredient varies depending on the its type, but, in general, it is contained in a sufficient amount for exhibiting a desired efficacy.

Examples of drugs which are used for treating the skin of a mammal and usable in the invention are as follows: drugs for parasitic skin disease, such as bifonazole, siccanin, bisdecalinium acetate, clotrimazole, and salicylic acid; drugs for suppurative disease, such as sulfamethoxazole sodium, erythromycin, and gentamicin sulfate; anti-inflammatory-analgesic agents such as indomethacin, ketoprofen, betamethasone valerate, and fluocinolone acetonide; antipruritic agents such as diphenhydramine; local anesthetics such as procaine hydrochloride and lidocaine hydrochloride; dermatologic-bacterioides, such as iodine, povidone-iodine, benzalkonium chloride, and chlorhexidine gluconate, and the like.

Examples of drugs to be applied on the coelom of mammals, i.e. on the rectum, the urethra, the nasal cavity, the vagina, the auditory meatus, the oral cavity, or the oral fassa and usable in the invention are as follows: antihistamic agents such as diphenhydramine hydrochloride and chlorpheniramine maleate; drugs for treating the genital organs, such as clotrimazole, naphazoline nitrate, ketotifen fumarate, and miconazole nitrate; otolaryngologic drugs such as tetryzoline hydrochloride; bronchodilators such as aminophylline; antimetabolites such as fluorouracin; hypnotic-sedative agents such as diazepam; antipyretic-analgesic-antiphlogistic agents such as aspirin, indomethacin, sulindac, phenylbutazone, and ibuprofen; adrenal hormone agents such as dexamethasone, triamcinolone, and hydrocortisone; local anesthetics such as lidocaine hydrochloride; drugs for suppurative diseases such as sulfisoxazole, kanamycin, tobramycin, and erythromycin; and synthetic antibiotics such as norfloxacin, and nalidixic acid; and the like.

A content of the active ingredient depends on the its type, but, in general, the content may preferably be in the range of about 0.001 to 10 wt.%.

Examples of a pH adjuster usable for the composition of the invention are acids such as hydrochloric acid, sulfuric acid, boric acid, phosphoric acid, and acetic acid; bases such as sodium hydroxide, monoethanolamine, diethanolamine, and triethanolamine; and the like.

The composition of the invention may contain a pharmaceutically acceptable buffer, salt, preservative, solubilizing agent, and the like, if necessary. Examples of the preservative are invert soaps such as benzalkonium chloride, benzethonium chloride, and chlorhexidine gluconate; parabenes such as methylparabene, ethylparabene, propylparabene, and butylparabene; alcohols such as chlorobutanol, phenylethylalcohol, and benzylalcohol; organic acids and salts thereof such as dehydroacetic acid sodium, sorbic acid, and sodium sorbate; and the like. Further, a surfactant or a chelating agent may be added, if necessary. The above ingredients may be used in an amount of about 0.001 to 2 wt.%, preferably about 0.002 to 1 wt.%. Examples of the buffer are alkali metal salts of acids such as phosphoric acid, boric acid, acetic acid, tartaric acid, lactic acid, and carbonic acid; amino acids such as glutamic acid, epsilon-aminocaproic acid, aspartic acid, glycyl, arginine, and lysine; taurine; trisaminomethane; and the like. The buffers may preferably be added in an amount required for maintaining a pH of the composition to 3 to 10.

Examples of the solubilizing agent are polysorbate 80, polyoxyethylene hydrogenated castor oil, cyclodextrin, and the like, and an amount of the solubilizing agent may preferably be in the range of 0.001 to 15 wt.%.

The second invention is an eyedrop enhanced ocular drug penetration owing to incorporating agar. Particularly, when the particulate agar is used as a vehicle of eyedrops, the eyedrops will have excellent characteristics. The invention also relates to an ocular drug penetration enhancer characterized by the use of agar as a vehicle.

Agar has already been used for foods and so forth and is easily obtained from seaweeds such as Gelidiales. Main constituents of agar are polysaccharides consisting of agarose and agaropectin, and agar is high in safety as is apparent from its insertion on Japanese Pharmacopoeia and is widely used for foods and the like. Further, it is possible to process agar so as to impart desired characteristics thereto, and it is relatively easy to modify agar physically or chemically. A water content of commercially available agar is usually from 10% to 30%, and the an eyedrop of the invention may be prepared by using the commercially available agar as it is or by using physically or chemically modified agar.

A production process of the agar to be used for the eyedrop of the invention is not limited, but it is preferred to use agar produced by an industrial process from the view point of stable supply. Examples of such agar are UP-6, UP-16, UP-37, M-7, M-9, AX-30, AX-100, AX-200, BX-30, BX-100, BX-200, PS-5, PS-6, PS-7, and PS-8, all of which are manufactured by Ina Food Industry Co., Ltd., and the like. The above agars usable in the invention can be used alone or in combination of two or more.

A molecular weight of the agar to be contained in the eyedrop of the invention is not particularly limited, but a weight-average molecular weight of the agar may preferably be from 5,000 to 1,200,000. A more preferable weight-average molecular weight may be from 30,000 to 800,000. When the weight-average molecular weight of agar is less than 5,000, ocular drug penetration is not so much enhanced. When the weight-average molecular weight of agar exceeds 1,200,000, it is difficult to maintain the viscosity of the eyedrops to 150 mPa•s or less. It is possible to measure the weight-average molecular weight of agar by using a gel permeation chromatography. An agar content in the eyedrop of the invention is not particularly limited but may preferably be from 0.1 to 10 wt.%. A more preferable agar content may be from 0.2 to 5 wt.%. When the agar content is less than 0.1 wt.%, ocular drug penetration is not so much enhanced. When the agar content exceeds 10 wt.%, the eyedrops may have a high viscosity and gelled in some cases.

From the view point of easy instillation, a viscosity measured by using an E type viscometer (25°C, shear rate: $100s^{-1}$) of the eyedrop of the invention may preferably be adjusted to 150 mPa•s (150 centipoises) or less. A more preferable viscosity of the eyedrop may be 100 mPa•s or less. When the viscosity of the eyedrop exceeds 150 mPa•s at instillation, it is difficult to apply the eyedrop in the state of droplets. Though it is possible to instill the eyedrop having a high viscosity by applying a larger force to the container, the eyedrop is inferior in flow-cutting and droplets are varied in amount to cause problems such as foreign-body sensation after instillation. It is also difficult to perform the mechanical sterilization which is generally performed for sterilizing eyedrops, when the viscosity is too high. By setting the viscosity in the above range, it is possible to maintain the amount of droplet constant and to impart a favorable feel in instillation. The viscosity of the eyedrop of the invention is measured by using the E type viscometer under the conditions of a measurement temperature of 25°C and a shear speed of $100s^{-1}$. In addition, when the eyedrop of the invention are used as an ophthalmic ointment, it is not problematic at all when a viscosity of the ointment is 150 mPa•s or more.

The agar to be contained in the eyedrop of the invention can be in any state of the agar contained in the liquid containing water as a maim component. For example, the agar may be dissolved either perfectly or partially into the liquid, or particles of the agar may be dispersed in the liquid. As used herein, the agar in the state of particles dispersed in the liquid means more specifically that particulate agar dispersed in water, and a particle diameter of the particulate agar is 100 $\mu$m or less. A more preferred particle diameter may be 20 $\mu$m or less, and a further preferred particle diameter may be 10 $\mu$m or less. When the agar particle diameter exceeds 100 $\mu$m, a storage stability of the eyedrop can be adversely affected, and problems such as foreign-body sensation after instillation may occur. A shape of the particulate agar is not particularly limited, and examples of the shape are spherical, elliptical, and other amorphous shapes.

The eyedrop of the invention can be prepared by adding active ingredient(s) to a liquid containing the particulate agar obtained by heating and dissolving agar in an aqueous solution followed by cooling under applying a stress.

The preparation process of the agar-containing liquid (agar-containing composition) is not particularly limited, but it is preferred that the aqueous solution containing agar is heated until the mixture becomes uniform and then the mixture is cooled gradually to a normal temperature with stirring vigorously so as to avoid gelling. In turn, it is possible to obtain the agar-containing liquid by pulverizing a gelled agar-containing composition under applying a stress such as a strong shear force.

A method of applying stress to the agar solution is not particularly limited, and examples of the method are vibration, shearing, stirring, compression, pulverizing, and the like. Among those, the stirring is most preferred as the method of applying stress to the agar solution. Any stirring appliances such as a magnetic stirrer, a mechanical stirrer, a mixer, a shaker, a rotor, and a homogenizer (T. K. HOMO MIXER. Tokushu Kika Kogyo Co., Ltd.) may be used or a manual stirring may be performed. It is desirable to use an apparatus which is capable of vigorous stirring in the preparation of the agar-containing liquid, and it is more desirable to perform the stirring with the Reynolds number of the stirring being maintained to 100 or more at a room temperature.

The agar-containing solution is heated at such a temperature that makes the agar-containing liquid to be visually uniform, and the heating temperature may preferably be 80°C or more, more preferably 95°C or more. Further, the agar-containing solution may be boiled when so required.

A method of cooling the agar-containing liquid may be any one of air-cooling, water-cooling, ice-cooling, solvent-cooling, wind-cooling, refrigeration, freezing, and the like. In the case of rapidly cooling the agar-containing liquid by the water-cooling, the ice-cooling, the refrigeration, the freezing, or the like, it is necessary to increase a stirring force in order to prevent the gelling.

The agar-containing solution having low viscosity and high concentration is prepared by the above method. Though the principle of the preparation has not been clearly defined, the following assumption is possible. Agar is gelled in such a manner that molecular chains of agar molecules form a hydrogen bonding to establish a helical structure, thereby incorporating a water molecule to establish a higher-order and stronger structure. After the agar is heated to a high temperature to become uniform, the agar molecules having the random coil molecular structure tend to form the helical structure as they are cooled. The application of the strong stress to the agar molecules during the cooling prevents the helical structure from being established, thereby enabling to obtain the agar solution which is not gelled and has a low viscosity.

Preferably, eyedrop of the invention contains agar having a weight-average molecular weight of from 5,000 to

1,200,000 and a particle diameter of 100 μm or less at a content of from 0.1 to 10 wt.% and has a viscosity of 150 mPa•s or less. More preferably, eyedrop of the invention contains agar having a weight-average molecular weight of from 30,000 to 800,000 and a particle diameter of 20 μm or less at a content of from 0.2 to 5 wt.% and has a viscosity of 100 mPa•s or less. Eyedrops obtained by incorporating a particulate agar having a particle diameter of 100 μm or less which is prepared by heating and dissolving agar having a weight-average molecular weight of from 5,000 to 1,200,000 in an aqueous solution and then cooling the agar solution under applying a stress is preferred since it is improved in ocular drug penetration. Moreover, eyedrops obtained by incorporating a particulate agar having a particle diameter of 20 μm or less which is prepared by heating and dissolving agar having a weight-average molecular weight of from 30,000 to 800,000 in an aqueous solution and then cooling the agar solution under applying a stress is more preferred since it is further enhanced ocular drug penetration.

Preferably, an ocular drug penetration enhancer of the invention is characterized by using as a vehicle the particulate agar obtained by heating and dissolving agar in an aqueous solution and then cooling the agar solution under applying a stress. More preferably, an ocular drug penetration enhancer of the invention uses as a vehicle the particulate agar having a particle diameter of 100 μm or less which is prepared by heating and dissolving agar having a weight-average molecular weight of from 5,000 to 1,200,000 in an aqueous solution and then cooling the agar solution under applying a stress. Further preferably, an ocular drug penetration enhancer of the invention uses, as a vehicle, the particulate agar having a particle diameter of 20 μm or less which is prepared by heating and dissolving agar having a weight-average molecular weight of from 30,000 to 800,000 in an aqueous solution and then cooling the agar solution under applying a stress.

A target disease of the eyedrop of the invention is not limited, and the eyedrop effectively works on diseases such as dry eye syndrome, glaucoma, cataract, inflammation, pollinosis, and so forth by containing the drug suitable for treating the disease.

Types of drugs which can be formulated to the eyedrop of the invention are not particularly limited, and examples of the drugs are antibacterial agents (quinolone-based antibacterial agents, cephalosporins, sulfacetamide sodium, sulfamethoxazole, etc.), anti-inflammatory agents (hydrocortisone, dexamethasone, prednisolone, betamethasone, diclofenac, indomethacin, fluorometholone, planoprofen, di-potassium glydyrrhizinate, epsilon-aminocaproic acid, etc.), antihistamic agents (chlorpheniramine maleate, diphenhydramine hydrochloride, etc.), antiglaucomic agents (prostaglandin derivatives, carbonate dehydrarase inhibitors, etc.), antiallergic agents (sodium cromoglycate, etc.), and the like. Examples of immunosuppressive agents and an time tabolites are methotrexate, cyclophosphamide, cyclosporine, 6-mercaptoprine, azathioprine, fluorouracil, tegafur, and the like. Mixtures of the above compounds such as antibiotic/anti-inflammatory agent mixtures including a combination of neomycin sulfate and dexamethasone sodium phosphate, etc. can also be formulated, and other drugs are usable depending on a symptom of the eye and treatment of the disease.

An amount of the drug to be formulated may preferably be in the range of 0.001 to 10 wt.%, but the amount is not particularly limited thereto so far as a curative effect is exhibited at the concentration.

Other additives such as an isotonic agent, a buffer, a pH adjuster, a solubilizing agent, a stabilizer, and a preservative may be formulated with the eyedrop of the invention as required.

Examples of the isotonic agent are glycerin, propyleneglycol, sodium chloride, potassium chloride, sorbitol, mannitol, and the like.

Examples of the buffer are phosphoric acid, phosphate, citric acid, acetic acid, epsilon-aminocaproic acid, trometamol, and the like.

Examples of the pH adjuster are hydrochloric acid, citric acid, phosphoric acid, acetic acid, sodiumhydroxide, potassium hydroxide, boric acid, borax, sodium carbonate, sodium hydrogencarbonate, and the like.

Examples of the solubilizing agent to be added when the drug or other additives is/are slightly soluble to water are polysorbate 80, polyoxyethylene hydrogenated castor oil 60, macrogol 4000, and the like.

Examples of the stabilizer are ethylenediamine tetraacetic acid, sodium ethylenediamine-tetraacetate, and the like.

Examples of the preservative are sorbic acid, potassium sorbate, benzalkonium chloride, benzethonium chloride, methylparabene, propylparabene, chlorobutanol, and the like, and the above preservatives may be used in combination.

The eyedrop of the invention can be prepared by adding agar and the drug to a sterilized pure water, and a viscosity of the eyedrop can be adjusted by high speed stirring, irradiation with ultrasonic rays, and the like to achieve a desired viscosity when so required.

As described in experiments later in this specification, fluorescein and pilocarpine were used as indexes for measuring an penetrated amount, and fluorescence of fluorescein penetrated to the cornea or the aqueous humor and a pupil diameter affected by pilocarpine were measured. Detailed results are described in embodiments, but, in the case of using the particulate agar of this invention as a vehicle, a remarkably superior ocular drug penetration is achieved as compared with controls. As is apparent from the experiments, the ocular drug penetration is enhanced by the use of the particulate agar as a vehicle.

It is preferred to set a pH of the eyedrop of the invention to 4.0 to 8.0, and an osmotic pressure may preferably be set to about 1.0.

An instillation frequency of the eyedrop of the invention can be selected properly depending on the symptom, the age of patients, the formulation of pharmaceutical compositions, and so forth, but it will be sufficient if the eyedrops are applied once to a several times per day.

Brief Description of the Drawings

Fig. 1 is an optical microscope photograph of an agar solution prepared by Example 1.

Fig. 2 is a graph showing a relationship between a viscosity and a stirring speed (equivalent to a shear speed) of each of Examples 4 to 9 and Examples 26 to 30.

Fig. 3 is a graph showing results of measurements of fluorescein concentrations in the cornea at the indicated time points after applying eyedrops of Experiments 10 and 12 and Comparative Experiment 2 (control) to rabbits. The vertical axis indicates the fluorescein concentration (ng/mL) in the cornea, and the horizontal axis indicates time (hr).

Fig. 4 is a graph showing results of measurements of fluorescein concentrations in the aqueous humor at the indicated time points after applying eyedrops of Experiments 10 and 12 and Comparative Experiment 2 (control) to rabbits. The vertical axis indicates the fluorescein concentration (ng/mL) in the aqueous humor, and the horizontal axis indicates time (hr).

Fig. 5 is a graph showing results of measurements of myosis by pilocarpine at the indicated time points after applying eyedrops of Experiment 20 and Comparative Experiment 3 (control) to rabbits. The vertical axis indicates the myosis by pilocarpine (mm), and the horizontal axis indicates time (hr).

Best Mode for Carrying out the Invention

The present invention will hereinafter be described through embodiments, but it should be understood that the embodiments are given only by way of example and not for the purpose of limiting the scope of the invention.

(1) Preparations and Evaluation Experiments of Agar-Containing Compositions (Agar Solutions)

1. Measurement of weight-average molecular weight

Weight-average molecular weights of agars were measured in accordance with the following measurement conditions.

Measurement Conditions

A. Apparatus: Gel Permeation Chromatography (product of Waters Co.),
(M510 type high pressure pump, U6 type universal injector).
B. Data processing: GPC data processing system which is a product of TRC (Toray Research Center).
C. Column: TSK-gel-GMPWXL (inner diameter: 7.8 mm, length: 30 cm) (two pieces) (product of Tosoh Corporation).
D. Solvent: 0.1 M-$NaNO_3$/distilled water
E. Flow rate: 1.0 ml/min.
F. Column temperature: 50˚C (column thermostat, Tosoh Corporation)
G. Sample
Concentration: 0.1% (wt/vol);
Solubility: confirmed by visual observation:
Filtration: 0.45 μm-Maishoridisuku W-13-5 (product of Tosoh Corporation).
H. Injection amount: 200 μl.
I. Detector: differential refractometer R-401 (product of Tosoh Corporation).
J: Molecular weight correction: 14 types of pullulans (products of Showa Denko K. K.).

The weight-average molecular weights of the agars measured under the above conditions are shown in Table 1. The agars shown in Table 1 are products of Ina Food Industry Co., Ltd.

Table 1

| Agar | Weight-Average Molecular Weight |
|---|---|
| AX-30 | 89,400 |
| AX-100 | 125,000 |
| AX-200 | 153,000 |
| BX-30 | 125,000 |
| BX-100 | 202,000 |
| BX-200 | 273,000 |
| UP-6 | 221,000 |
| M-9 | 449,800 |
| PS-7 | 374,000 |
| Agarose (agarose DNA grade) | 299,000 |

2. Particle diameter measurement method

After preparing agar-containing compositions, particles of each of the compositions were observed by using an optional microscope (OPTIPHOTO-2, Nikon Corporation) to detect a maximum particle diameter of each of the compositions.

3. Viscosity measurement method

After preparing the agar-containing compositions, a viscosity of each of the compositions was measured by using a B type viscometer (rotor No. 2) under the conditions of 20˚C and 60 rpm.

4. Preparation process

Process A

Agar was placed in a 500 ml flask, and then a distilled water was poured into the flask until a weight reached 500 g. The mixture was heated to about 100˚C to dissolve the agar and then cooled to 20˚C with stirring at 1,500 rpm using a magnetic stirrer.

Process B

Agar was placed in a 500 ml flask, and then a distilled water was poured into the flask until a weight reached 500 g. The mixture was heated using a microwave oven to a high temperature to dissolve the agar and then cooled to 20˚C with stirring at 1,500 rpm using a magnetic stirrer. After that, 13 g of glycerin was added to the mixture, followed by stirring at 20˚C and 1,500 rpm using a magnetic stirrer for 30 minutes. Process C

Agar was placed in a 500 ml four neck flask with a Teflon stirring blade, and then a distilled water was poured into the flask until a weight reached 500 g. The mixture was heated in an oil bath to about 100˚C to dissolve the agar and then cooled to 20˚C with stirring at 700 rpm.

Process D

Agar was placed in a 500 ml four neck flask with a Teflon stirring blade, and then a distilled water was poured into the flask until a weight reached 500 g. The mixture was heated in an oil bath to about 100˚C to dissolve the agar and then cooled to 80˚C with stirring at 700 rpm using the stirring blade. After transferring the solution to a stainless vessel, the solution was cooled to 20˚C with stirring using a homomixer (T. K. Homo Mixer, Tokushu Kika Kogyo Co., Ltd.).

5. Example

Examples of the agar-containing compositions (agar solutions) of the invention are described below. Each of the agar solutions obtained by Examples 1 to 25 had a viscosity of 700 mPa•s or less, and particulate agar was found in the solution by an observation using an optical microscope. An optical microscope photograph of the agar solution of Example 18 is shown in Fig. 1.

Example 1

An agar-containing composition (agar solution) was prepared by using 2.5 g (0.5 wt.%) of agar (UP-6) through the preparation process A. The obtained agar solution was opaque and had a viscosity of 93 mPa•s and a particle diameter of less than 10 μm.

Example 2

An agar-containing composition (agar solution) was prepared by using 2.5 g (0.5 wt.%) of agar (AX-30) through the preparation process A. The obtained agar solution was opaque and had a viscosity of 40 mPa•s and a particle diameter of less than 10 μm.

Example 3

An agar-containing composition (agar solution) was prepared by using 2.5 g (0.5 wt.%) of agar (AX-30) through the preparation process B. The obtained agar solution was opaque and had a viscosity of 31 mPa•s.

Example 4

An agar-containing composition (agar solution) was prepared by using agar (AX-30) through the preparation process D with the number of revolutions of the homomixer being set to 3,000 rpm. The obtained agar solution was pale yellow and opaque and had a viscosity of 32 mPa•s and a particle diameter of less than 10 μm.

Example 5

An agar-containing composition (agar solution) was prepared by using agar (AX-30) through the preparation process D with the number of revolutions of the homomixer being set to 4, 000 rpm. The obtained agar solution was pale yellow and opaque and had a viscosity of 51 mPa•s and a particle diameter of less than 10 μm.

Example 6

An agar-containing composition (agar solution) was prepared by using agar (AX-30) through the preparation process D with the number of revolutions of the homomixer being set to 5.000 rpm. The obtained agar solution was pale yellow and opaque and had a viscosity of 67 mPa•s and a particle diameter of less than 10 μm.

Example 7

An agar-containing composition (agar solution) was prepared by using agar (AX-30) through the preparation process D with the number of revolutions of the homomixer being set to 6,000 rpm. The obtained agar solution was pale yellow and opaque and had a viscosity of 81 mPa•s and a particle diameter of less than 10 μm.

Example 8

An agar-containing composition (agar solution) was prepared by using agar (AX-30) through the preparation process D with the number of revolutions of the homomixer being set to 8,000 rpm. The obtained agar solution was pale yellow and opaque and had a viscosity of 94 mPa•s and a particle diameter of less than 10 μm.

Example 9

An agar-containing composition (agar solution) was prepared by using agar (AX-30) through the preparation process D with the number of revolutions of the homomixer being set to 10,000 rpm. The obtained agar solution was

pale yellow and opaque and had a viscosity of 144 mPa•s and a particle diameter of less than 10 μm.

Example 10

An agar-containing composition (agar solution) was prepared by using 2.5 g (0.5 wt.%) of agar (UP-6) through the preparation process D with the number of revolutions of the homomixer being set to 6,000 rpm. The obtained agar solution was opaque and had a viscosity of 403 mPa•s and a particle diameter of less than 10 μm.

Example 11

An agar-containing composition (agar solution) was prepared by using 2.5 g (0.5 wt.%) of agar (M-9) through the preparation process D with the number of revolutions of the homomixer being set to 6,000 rpm. The obtained agar solution was opaque and had a viscosity of 200 mPa•s and a particle diameter of less than 10 μm.

Example 12

An agar-containing composition (agar solution) was prepared by using 2.5 g (0.5 wt.%) of agar (AX-30) through the preparation process D with the number of revolutions of the homomixer being set to 6,000 rpm. The obtained agar solution was pale yellow and opaque and had a viscosity of 20 mPa•s and a particle diameter of less than 10 μm.

Example 13

An agar-containing composition (agar solution) was prepared by using 2.5 g (0.5 wt.%) of agar (AX-100) through the preparation process D with the number of revolutions of the homomixer being set to 6,000 rpm. The obtained agar solution was pale yellow and opaque and had a viscosity of 62 mPa•s and a particle diameter of less than 10 μm.

Example 14

An agar-containing composition (agar solution) was prepared by using 2.5 g (0.5 wt.%) of agar (AX-200) through the preparation process D with the number of revolutions of the homomixer being set to 6,000 rpm. The obtained agar solution was pale yellow and opaque and had a viscosity of 62 mPa•s and a particle diameter of less than 10 μm.

Example 15

An agar-containing composition (agar solution) was prepared by using 2.5 g (0.5 wt.%) of agar (BX-30) through the preparation process D with the number of revolutions of the homomixer being set to 6,000 rpm. The obtained agar solution was pale yellow and opaque and had a viscosity of 96 mPa•s and a particle diameter of less than 10 μm.

Example 16

An agar-containing composition (agar solution) was prepared by using 2.5 g (0.5 wt.%) of agar (agarose: agarose DNA grade) through the preparation process D with the number of revolutions of the homomixer being set to 6,000 rpm. The obtained agar solution was opaque and had a viscosity of 158 mPa•s and a particle diameter of less than 10 μm.

Example 17

An agar-containing composition (agar solution) was prepared by using 2.5 g (0.5 wt.%) of agar (PS-7) through the preparation process D with the number of revolutions of the homomixer being set to 6,000 rpm. The obtained agar solution was opaque and had a viscosity of 190 mPa•s and a particle diameter of less than 10 μm.

Example 18

An agar-containing composition (agar solution) was prepared by using 5.0 g (1.0 wt.%) of agar (UP-6) through the preparation process A. The obtained agar solution was opaque and had a viscosity of 384 mPa•s and a particle diameter of less than 20 μm.

Example 19

An agar-containing composition (agar solution) was prepared by using 5.0 g (1.0 wt.%) of agar (trade name: BA-10, Funakoshi Co., Ltd.) through the preparation process A. The obtained agar solution was opaque and had a viscosity of 297 mPa•s and a particle diameter of less than 20 μm.

Example 20

An agar-containing composition (agar solution) was prepared by using 2.5 g (0.5 wt.%) of agar (AX-100) through the preparation process A. The obtained agar solution was opaque and had a viscosity of 133 mPa•s and a particle diameter of less than 10 μm.

Example 21

An agar-containing composition (agar solution) was prepared by using 2.5 g (0.5 wt.%) of agar (AX-200) through the preparation process A. The obtained agar solution was opaque and had a viscosity of 133 mPa•s and a particle diameter of less than 10 μm.

Example 22

An agar-containing composition (agar solution) was prepared by using 2.5 g (0.5 wt.%) of agar (BX-30) through the preparation process A. The obtained agar solution was opaque and had a viscosity of 41 mPa•s and a particle diameter of less than 10 μm.

Example 23

An agar-containing composition (agar solution) was prepared by using 2.5 g (0.5 wt.%) of agar (BX-30) through the preparation process C. The obtained agar solution was opaque and had a viscosity of 142 mPa•s and a particle diameter of less than 10 μm.

Example 24

An agar-containing composition (agar solution) was prepared by using 2.5 g (0.5 wt.%) of agar (PS-7) through the preparation process A. The obtained agar solution was opaque and had a viscosity of 87 mPa•s and a particle diameter of less than 10 μm.

Example 25

An agar-containing composition (agar solution) was prepared by using 2.5 g (0.5 wt.%) of agar (M-9) through the preparation process A. The obtained agar solution was opaque and had a viscosity of 106 mPa•s and a particle diameter of less than 10 μm.

Example 26

A gellan gum solution was prepared in the same manner as in Example 4 except for using gellan gum (GP-10, Ina Food Industry Co., Ltd.) in place of agar. The obtained solution was transparent and had a viscosity of 137 mPa•s and a particle diameter of less than 10 μm.

Example 27

A gellan gum solution was prepared in the same manner as in Example 6 except for using gellan gum (GP-10, Ina Food Industry Co., Ltd.) in place of agar. The obtained solution was transparent and had a viscosity of 160 mPa•s and a particle diameter of less than 10 μm.

Example 28

A gellan gum solution was prepared in the same manner as in Example 7 except for using gellan gum (GP-10, Ina Food Industry Co., Ltd.) in place of agar. The obtained solution was transparent and had a viscosity of 150 mPa•s

and a particle diameter of less than 10 μm.

Example 29

A gellan gum solution was prepared in the same manner as in Example 8 except for using gellan gum (GP-10, Ina Food Industry Co., Ltd.) in place of agar. The obtained solution was transparent and had a viscosity of 148 mPa•s and a particle diameter of less than 10 μm.

Example 30

A gellan gum solution was prepared in the same manner as in Example 9 except for using gellan gum (GP-10, Ina Food Industry Co., Ltd.) in place of agar. The obtained solution was transparent and had a viscosity of 158 mPa•s and a particle diameter of less than 10 μm.

Example 31

A gellan gum solution was prepared by using gellan gum (GP-10, Ina Food Industry Co., Ltd.) in place of agar and through the preparation process D with the number of revolutions of the homomixer being set to 6,000 rpm. The obtained solution was transparent and had a viscosity of 103 mPa•s and a particle diameter of less than 10 μm.

Comparative Example 1

2.5 g (0.5 wt.%) of agar (UP-6) was placed in a 500ml flask, and 500 g of distilled water was poured into the flask to disperse the agar. The agar was dissolved at a high temperature and then cooled to 20°C without stirring. The obtained 0.5 wt.% agar gel was filtered using a sieve having a pore size of ϕ1 mm. A viscosity of the pulverized gel was 900 mPa•s.

Comparative Example 2

An agar solution was prepared by using 0.25 g (0.05 wt.%) of agar (AX-30) through the preparation process A. The obtained agar solution had a viscosity of 14 mPa•s.

Comparative Example 3

An agar solution was prepared using 2.5 g (0.5 wt.%) of agar (AX-30) through the preparation process A except for setting the number of revolutions of a magnetic stirrer to 500 rpm. The obtained agar solution was a mixture of a gel and a solution, and it was impossible to detect a viscosity thereof.

6. Application evaluation

Ease of application and a state after application in the case of applying each of Example 1 to 3 and Comparative Examples 1 to 3 on the human skin were evaluated. Results of the evaluations are shown in Table 2.

Table 2

| | Ease of Application | State after Application |
|---|---|---|
| Ex. 1 | easy | hardly streaming down |
| Ex. 2 | easy | hardly streaming down |
| Ex. 3 | easy | hardly streaming down |
| Comp. Ex. 1 | difficult | hardly streaming down |
| Comp. Ex. 2 | easy | easily streaming down |
| Comp. Ex. 3 | difficult | hardly streaming down |

From Table 2, it is apparent that the solutions of Examples 1 to 3 have the characteristics of easy application and hardly streamingdown. On the other hand, the solutions of Comparative Examples 1 and 3 are difficult to be applied,

though they are hardly streaming down. The solution of Comparative Example 2 is easily applied but has a drawback of easily streaming down.

7. Relationship between viscosity and stirring speed

Shown in Fig. 2 is a relationship between the viscosity and the stirring speed (corresponding to the shear speed) of each of Examples 4 to 9 and 26 to 30. As is apparent from Fig. 2, the viscosity is increased with the increase in the stirring speed in Examples 4 to 9, while the viscosity is almost constant even when the stirring speed is increased in Examples 26 to 30.

8. Viscosity ratio evaluation experiment

To each of the agar solutions (50 g) of Examples 10 to 17 and 31, 0.025 g of fluorescein sodium and 1.3 g of glycerin, both of which are water-soluble compounds, were added. After mixing each of the agar solutions sufficiently, a pH of each of the agar solutions was adjusted to pH7. A viscosity of each of the agar solutions was measured by using the B type viscometer (rotor No. 2) under the conditions of 20˚C and 60 rpm. The thus-measured viscosity is represented by Y.

Next, NaCl was added to each of the agar solutions to adjust a concentration to 0.9 wt.%, followed by sufficient mixing. A viscosity of each of the agar solutions was measured by using the B type viscometer (rotor No. 2) under the conditions of 20˚C and 60 rpm. The thus-measured viscosity is represented by Z.

The viscosity ratio X was calculated based on the following equation:

$$X = Z/Y.$$

Each of the solutions was applied on the human fore-arm skin to compare the solutions with one another in terms of ease of application and a state after application. Results of the experiments are shown in Table. 3.

Table 3

| | Agar Solution | X (viscosity Ratio) | Y (mPa•s) | Z (mPa•s) | Ease of Application *1 | State of Application *2 |
|---|---|---|---|---|---|---|
| Exp. 1 | Ex. 10 | 1.019 | 403 | 411 | ○ | ○ |
| Exp. 2 | Ex. 11 | 1.338 | 200 | 268 | ○ | ○ |
| Exp. 3 | Ex. 12 | 1.175 | 20 | 24 | ○ | ○ |
| Exp. 4 | Ex. 13 | 1.724 | 62 | 106 | ○ | ○ |
| Exp. 5 | Ex. 14 | 1.260 | 62 | 78 | ○ | ○ |
| Exp. 6 | Ex. 15 | 1.422 | 96 | 137 | ○ | ○ |
| Exp. 7 | Ex. 16 | 1.013 | 158 | 160 | ○ | ○ |
| Exp. 8 | Ex. 17 | 1.276 | 190 | 243 | ○ | ○ |
| Exp. 9 | Ex. 31 | 3.259 | 103 | 334 | ○ | △ |
| Comp. Exp. 1 | Pure Water | 1.000 | 1 | 1 | ○ | × |

*1 ease of application
○: good, ×: poor
*2 state of application
○: hardly streaming down after application
△: hardly streaming down but gives a slight unpleasant feel
×: easily streaming down or a great unpleasant feel

As is apparent from Table 3, in Experiments 1 to 8, the characteristics of ease of application, appropriate viscosity on the skin surface, and hardly streaming down were exhibited. On the contrary, in Experiment 9 and Comparative Experiment 1, the ease of application was achieved, but, in Experiment 9, unpleasant feel was caused because the viscosity was increased rapidly after application to make the solution gelled on the skin surface. The drawback of easily streaming down after application was detected in Comparative Experiment 1.

(2) Ocular pharmacokinetic study Ocular pharmacokinetic study

A. Ocular pharmacokinetic study by fluorophotometry method

(1) Preparation of sample eyedrop

To each of the agar solutions (100 g) prepared by the Examples 1, 2, and 18 to 25, 0.05 g of fluorescein sodium and 2.6 g of concentrated glycerin were added. Each of the mixtures was stirred for 2 minutes using a hybrid mixer (HM-500, Keyence Corporation) to obtain sample eyedrops. A pH of each of the sample eyedrops was adjusted to 7. 0 (±0.5) by adding thereto sodium hydroxide or diluted hydrochloric acid, and then a viscosity of each of the sample eyedrops was measured by using an E type viscometer (Rotovisco CV20, Thermo Haake GmbH) under the conditions of 25˚C and a shear speed of $100s^{-1}$. A maximum particle size of each of agars was measured by using an optical microscope (OPTIPHOTO-2, Nikon Corporation). As a control, a comparative eyedrop (Comparative Experiment 2) was obtained in the same manner as described above except for using a sterilized pure water in place of the agar solutions.

(ii) Administration method and measurement method

After administering the sample eyedrops obtained by the above preparation methods to the eyes of male Japanese white rabbits, fluorescein concentrations of 1, 2, 3, 4, 6, and 8 hours after the administrations in the cornea and the aqueous humor were measured by using a fluorophotometer (four measurements were conducted for each of the sample eyedrops and the comparative eyedrop to calculate an average value of the four measurements). An AUC (area under concentration curve) was calculated from the measurement values of the fluorescein concentrations, and an AUC ratio of each of the sample eyedrops to the comparative eyedrop was obtained by the following equation. Results of the experiments are shown in Table 4. Transitions of the fluorescein concentrations in the cornea and the aqueous humor are shown in Figs. 3 and 4.

AUC ratio = [AUC after sample eyedrops administration (ng•hr/mL)]/[AUC after comparative eyedrops administration (ng•hr/mL)]

In addition, an eyedrop was prepared in the same manner as described above except for using a CVP solution (1.0%) in place of the agar solutions , but it was impossible to drop the eyedrop containing CVP into the eye due to its very high viscosity (1,139 mPa•s).

Table 4

| | Agar Solution (wt.%) | Viscosity (mPa•s) | Particle diameter (μm) | AUC ratio (cornea) | AUC ratio (aqueous humor) |
|---|---|---|---|---|---|
| Exp. 10 | Ex. 18 (1.0%) | 23 | less than 20 | 3.8 | 4.7 |
| Exp. 11 | Ex. 1 (0.5%) | 32 | less than 10 | | 2.9 |
| Exp. 12 | Ex. 19 (1.0%) | 112 | less than 20 | 3.8 | 4.6 |
| Exp. 13 | Ex. 2 (0.5%) | 16 | less than 10 | | 5.7 |
| Exp. 14 | Ex. 20 (0.5%) | 28 | less than 10 | | 4.6 |
| Exp. 15 | Ex. 21 (0.5%) | 30 | less than 10 | | 4.8 |
| Exp. 16 | Ex. 22 (0.5%) | 11 | less than 10 | | 3.1 |
| Exp. 17 | Ex. 23 (0.5%) | 26 | less than 10 | | 5.4 |

(continued)

| | Agar Solution (wt.%) | Viscosity (mPa•s) | Particle diameter (μm) | AUC ratio (cornea) | AUC ratio (aqueous humor) |
|---|---|---|---|---|---|
| Exp. 18 | Ex. 24 (0.5%) | 8 | less than 10 | | 2.8 |
| Exp. 19 | Ex. 25 (0.5%) | 34 | less than 10 | | 3.9 |
| Comp. Exp. 2 | pure water | | | 1 | 1 |

B. Ocular pharmacological study using pilocarpine

(i) Preparation method of sample eyedrop

To the agar solution (100 g) prepared by Example 1, 1. 0 g of pilocarpine hydrochloride and 2.6 g of concentrated glycerin were added. Next, the solution was stirred using a magnetic stirrer to obtain a sample eyedrop. Then, a pH of the sample eyedrop was adjusted to 7.0 by adding thereto sodium hydroxide and diluted hydrochloric acid. A viscosity of the sample eyedrop was measured by using the E type viscometer (25˚C). As a control, a comparative eyedrop (Comparative Experiment 3) was obtained in the same manner except for using a sterilized pure water in place of the agar solution (Example 1).

(ii) Administration method and measurement method

After administering the sample eyedrops obtained by the above preparation methods to the eyes of male Japanese white rabbits, pupil diameters of 0.25, 0.5, 0.75, 1, 1.5, 2, 2.5, and 3 hours after the administration were measured. Myosis values were obtained by subtracting each of the pupil diameters at the above time points from a pupil diameter before the administration (six measurements were conducted for each of the sample eyedrop and the comparative eyedrop to calculate an average value of the six measurements). Further, an AUC (area under efficacy-time curve) was calculated from the obtained myosis values. Results of the experiments are shown in Table 5 and Fig. 5.

Table 5

| | Agar Solution (wt%) | Viscosity (mPa•s) | AUC (mm•hr) |
|---|---|---|---|
| EXp. 20 | Ex. 1 (0.5%) | 38 | 12.2 |
| Comp. Exp. 3 | pure water | | 5.9 |

(3) Formulation Example

Formulation examples of representative eyedrops and eardrops of the invention are as follows.

Formulation Example 1

Pilocarpine hydrochloride and concentrated glycerin were added to the agar solution (100 g) prepared by Example 18, and the mixture was stirred for 2 minutes using a hybrid mixer (HM-500, Keyence Corporation). Then, a pH of the mixture was adjusted to 7.0 by adding thereto 0.1 N sodium hydroxide or 0.1 N diluted hydrochloric acid to prepare an eyedrop.
in 100 g of the eyedrop:

| | |
|---|---|
| agar (UP-6) | 1.0 g |
| pilocarpine hydrochloride | 1.0 g |
| concentrated glycerin | 2.6 g |
| sodium hydroxide | quantum sufficient |
| hydrochloric acid | quantum sufficient |
| sterilized pure water | quantum sufficient |

Formulation Example 2

Planoprofen and concentrated glycerin were added to the agar solution (100 g) prepared by Example 1, and the mixture was stirred for 2 minutes using a hybrid mixer (HM-500. Keyence Corporation). Then, a pH of the mixture was adjusted to 7.0 by adding thereto 0.1 N sodium hydroxide or 0.1 N diluted hydrochloric acid to prepare an eyedrop. in 100 g of the eyedrop:

| | |
|---|---|
| agar (UP-6) | 0.5 g |
| planoprofen | 0.1 g |
| concentrated glycerin | 2.6 g |
| sodium hydroxide | quantum sufficient |
| hydrochloric acid | quantum sufficient |
| sterilized pure water | quantum sufficient |

It is possible to prepare eyedrops each containing agar at a desired concentration by preparing agar solutions of various concentrations and performing the operation same as that of Formulation Examples 1 and 2.

Formulation Example 3

An eardrop was prepared in accordance with the following formulation.

| | |
|---|---|
| gatifloxacin | 0.5 g |
| sodium ethylenediamine-tetraacetate | 0.1 g |
| sodium chloride | 0.9 g |
| agar solution of Example 12 | 70.0 g |
| pure water | 28.5 g |

The mixture of the above ingredients was stirred until the mixture became uniform, and then a pH of the thus-prepared solution was adjusted to 7.0 by adding thereto an optimum amount of hydrochloric acid or sodium hydroxide. The obtained eardrop was excellent in spreading property though it had a low viscosity. The eardrop was free from streaming down after administration.

Industrial Applicability

The present invention enables a preparation of a composition containing polysaccharide at a high concentration and yet remaining in the state of a liquid having a low viscosity by heating polysaccharide in a water-containing liquid and then cooling the mixture under applying a shear force as well as a provision of drugs, foods, cosmetics, toiletry products, and the like having a novel texture and/or function. In the case of using polysaccharide as bowel movement-improving foods, the invention enables to provide the bowel movement-improving foods as liquid foods, not in the state of a jelly, which are much more easily ingested.

As is apparent from the results of the Ocular pharmacokinetic studies using the fluorophotometry (see Table 4, Figs. 3 and 4), the eyedrop of the invention has the viscosity suitable for eyedrops and, with the instillation thereof, fluorescein is delivered to the cornea and the aqueous humor to remain there at the high concentration for a long time (3 to 6 times that of the control). As is apparent from the results of the Ocular pharmacological studies using pilocarpine which is one of the ophthalmic drugs (see Table 5 and Fig. 5), with the instillation of the eyedrop of the invention, the pilocarpine AUC (area under pharmaceutical effect-time curve) is increased to twice that of the control. Thus, the agar-containing eyedrop of the invention exhibits the effect of enhancing ocular drug penetration, and, owing to the low viscosity of the eyedrops, the easy instillation, the constant droplet amount, and the pleasant feel in dropping are achieved.

## Claims

1. An eyedrop comprising agar as an enhancer for ocular drug penetration.

2. The eyedrop according to claim 1, **characterized in that** the agar has a weight-average molecular weight of from 5,000 to 1,200,000.

3. The eyedrop according to claim 1 or 2, **characterized in that** the agar has a weight-average molecular weight of

from 30,000 to 800,000.

4. The eyedrop according to any one of claims 1 to 3, **characterized in that** the agar content is from 0.1 to 10 wt.%.

5. The eyedrop according to any one of claims 1 to 3, **characterized in that** the agar content is from 0.2 to 5 wt.%.

6. The eyedrop according to any one of claims 1 to 5, **characterized in that** the eyedrop has a viscosity measured by using an E type viscometer (25˚C, shear speed of $100s^{-1}$) of 150 mPa•s or less.

7. The eyedrop according to any one of claims 1 to 5, **characterized in that** the eyedrop has a viscosity measured by using an E type viscometer (25˚C, shear speed of $100s^{-1}$) of 100 mPa•s or less.

8. The eyedrop according to any one of claims 1 to 7, **characterized in that** the agar is in the state of particles and has the particle diameter of 100 μm or less.

9. The eyedrop according to any one of claims 1 to 7, **characterized in that** the agar is in the state of particles and has the particle diameter of 20 μm or less.

10. An eyedrop according to any one of claims 1, 2, 4, 6 and 8, comprising 0.1 to 10 wt.% of agar, the said agar having a weight-average molecular weight of from 5,000 to 1,200,000 and being in the state of particles with a diameter of 100 μm or less, the eyedrop having a viscosity measured by using an E type viscometer (25˚C, shear speed of $100s^{-1}$) of 150 mPa•s or less.

11. An eyedrop according to any one of claims 1, 3, 5, 7 and 9, comprising 0.2 to 5 wt.% of agar, the said agar having a weight-average molecular weight of from 30,000 to 800,000 and being in the state of particles with a diameter of 20 μm or less, the eyedrop having a viscosity measured by using an E type viscometer (25˚C, shear speed of $100s^{-1}$) of 100 mPa•s or less.

12. An eyedrop enhanced ocular drug penetration according to any one of claims 1 to 11, **characterized in that** it comprises a particulate agar-containing composition, the said composition being obtained by dissolving agar into an aqueous solution, by heating and then cooling the mixture under applying a stress.

13. The eyedrop according to claim 12, **characterized in that** the agar has a weight-average molecular weight of from 5,000 to 1,200,000 and the particulate agar has a particle diameter of 100 μm or less.

14. The eyedrop according to claim 12, **characterized in that** the agar has a weight-average molecular weight of from 30,000 to 800,000 and the particulate agar has a particle diameter of 20 μm or less.

15. Use of agar as an enhancer for the preparation of an eyedrop according to any one of claims 1 to 14.

16. Use of agar according to 15 in an effective amount for the preparation of an eyedrop intended to treat eye diseases in a patient.

Fig. 1

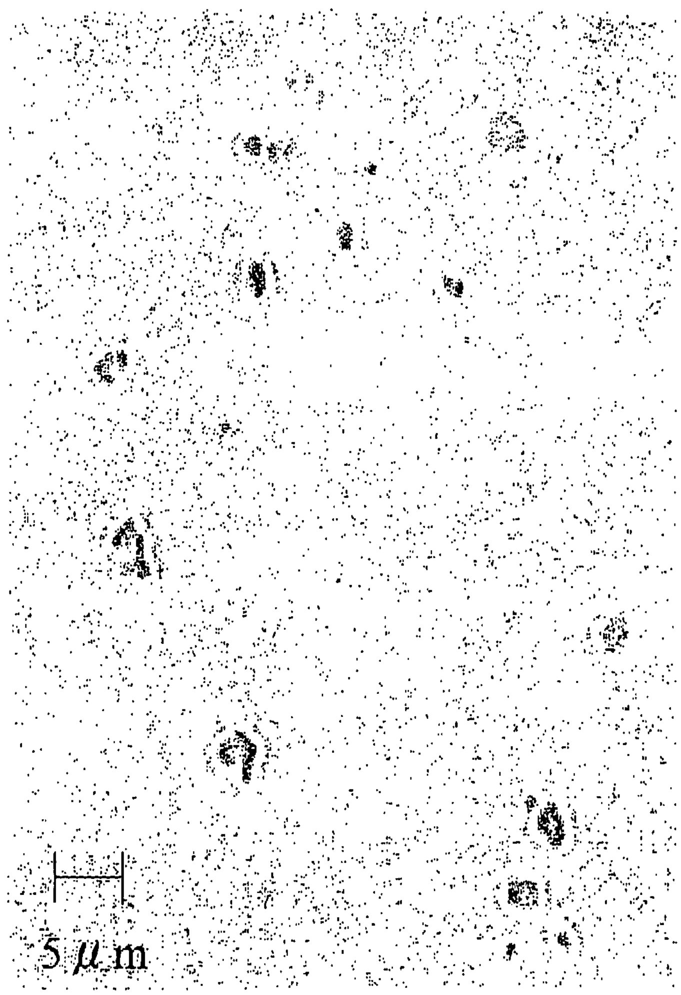

Fig. 2

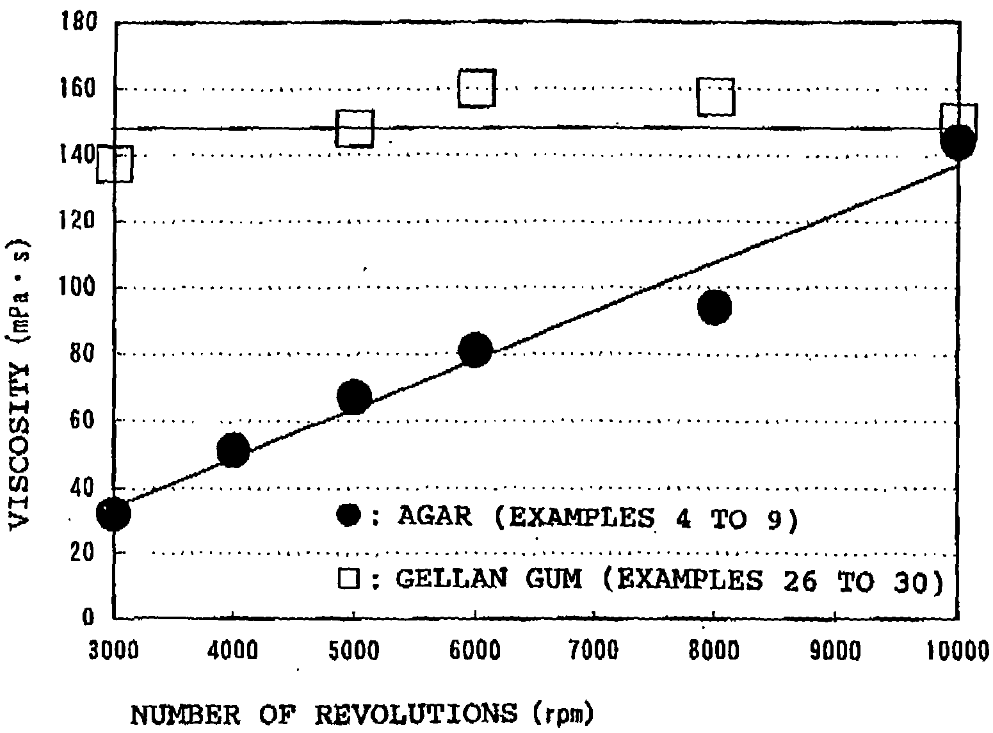
180
160
140
120
100
80
60
40
20
0
VISCOSITY (mPa・s)
3000
4000
5000
6000
7000
8000
9000
10000
NUMBER OF REVOLUTIONS (rpm)
●: AGAR (EXAMPLES 4 TO 9)
□: GELLAN GUM (EXAMPLES 26 TO 30)

Fig. 5

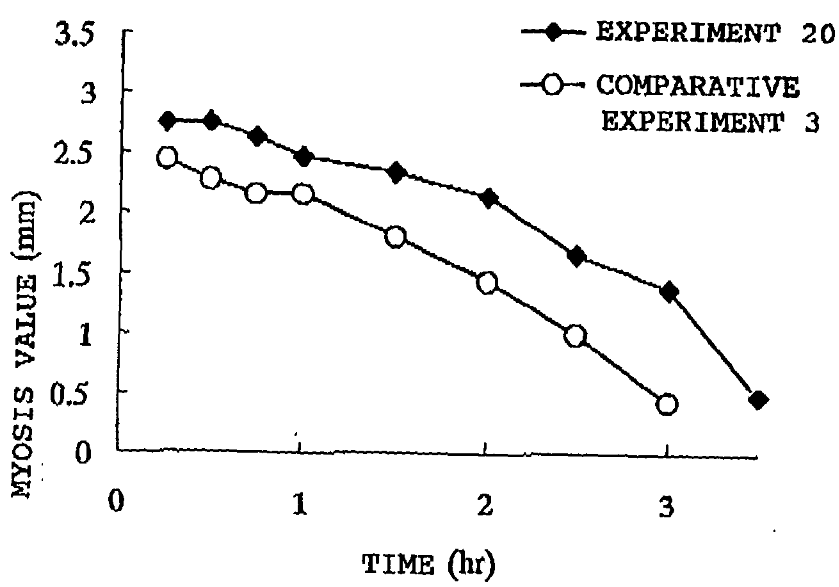
3.5
3
2.5
2
1.5
1
0.5
0
MYOSIS VALUE (mm)
0
1
2
3
TIME (hr)
EXPERIMENT 20
COMPARATIVE EXPERIMENT 3

Fig. 3

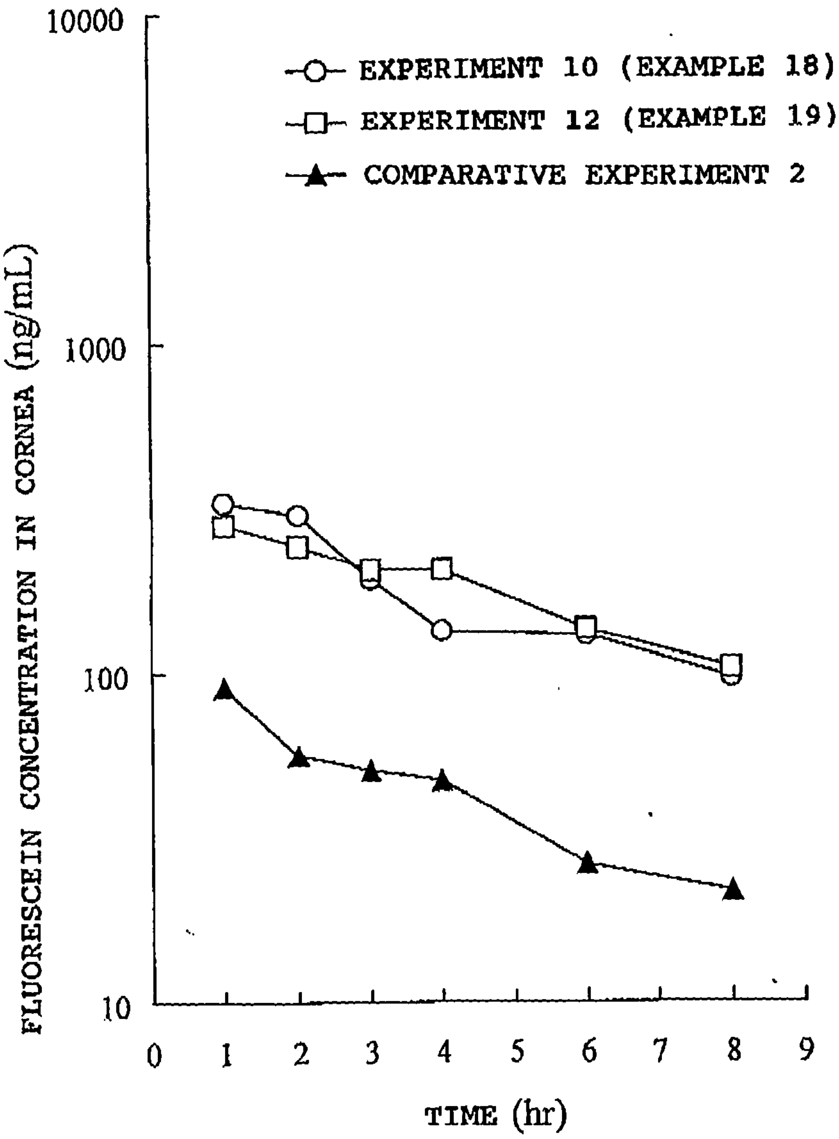
10000
1000
100
10
FLUORESCEIN CONCENTRATION IN CORNEA (ng/mL)
-○- EXPERIMENT 10 (EXAMPLE 18)
-□- EXPERIMENT 12 (EXAMPLE 19)
-▲- COMPARATIVE EXPERIMENT 2
0
1
2
3
4
5
6
7
8
9
TIME (hr)

Fig. 4

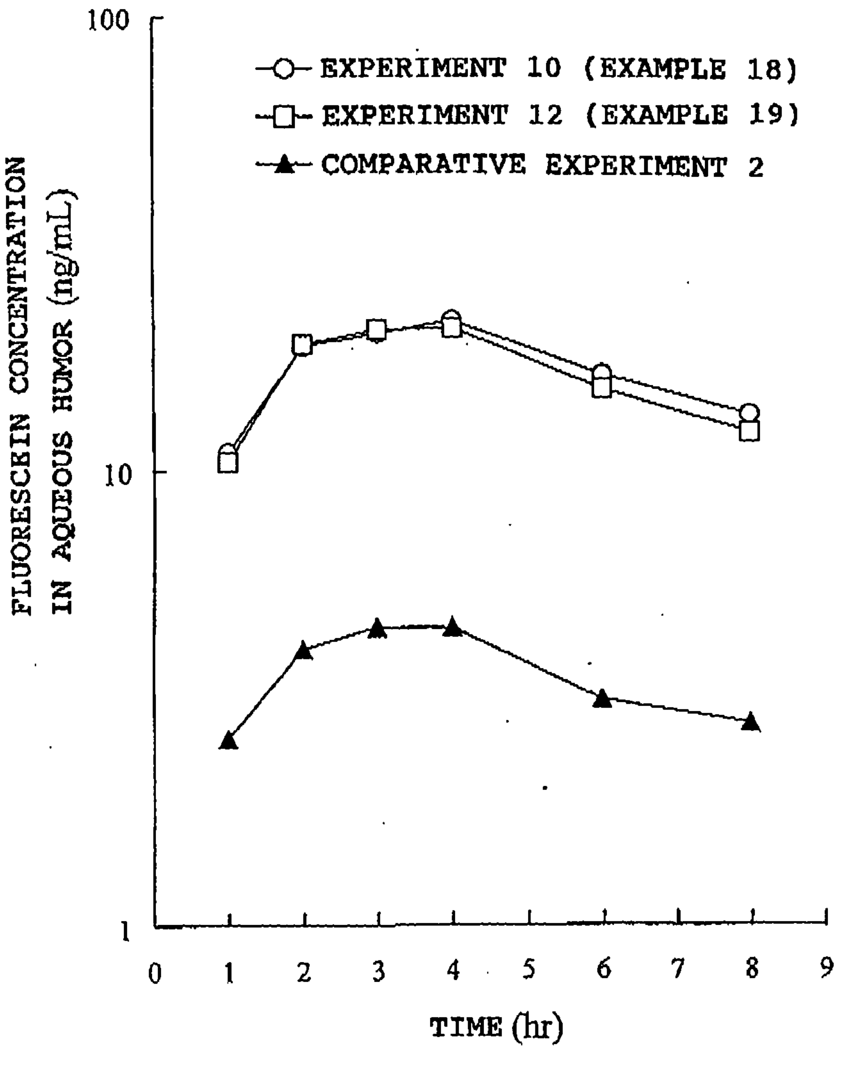
100
10
1
FLUORESCEIN CONCENTRATION
IN AQUEOUS HUMOR (ng/mL)
EXPERIMENT 10 (EXAMPLE 18)
EXPERIMENT 12 (EXAMPLE 19)
COMPARATIVE EXPERIMENT 2
0
1
2
3
4
5
6
7
8
9
TIME (hr)

Europäisches Patentamt
European Patent Office
Office européen des brevets

# EUROPEAN SEARCH REPORT

Application Number
EP 10 00 2999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 0 507 224 A2 (ALCON LAB INC [US]) 7 October 1992 (1992-10-07)<br>* page 2, lines 3-6, 51-58 *<br>* page 4, lines 25-33; examples 1-29; table 1 *<br>* page 21, line 54 - page 22, line 41; claims 1-18; figures 1a-1d *<br>----- | 1-16 | INV.<br>A61K47/36<br>A61K9/08<br>A61K47/10<br>A61P17/00<br>A61P27/04<br>C08B37/12<br>C08J3/03<br>A23L1/0532<br>A61K9/06<br>A23L1/0526 |
| Y | EP 0 355 908 A1 (UNILEVER NV [NL]; UNILEVER PLC [GB]) 28 February 1990 (1990-02-28)<br>* page 2, lines 1-5 *<br>* page 4, line 27; examples 1-3 *<br>----- | 1-16 | |
| Y | US 5 212 162 A (MISSEL PAUL J T [US] ET AL) 18 May 1993 (1993-05-18)<br>* column 1, lines 10-20, 55-68, *<br>* column 4, lines 36-48; claims 1-27; examples 1-3 *<br>----- | 1-16 | |
| | | | TECHNICAL FIELDS SEARCHED (IPC) |
| | | | A61K<br>A23L |

The present search report has been drawn up for all claims

1

| Place of search | Date of completion of the search | Examiner |
|---|---|---|
| Munich | 21 June 2010 | Toulacis, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 00 2999

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2010

| Patent document cited in search report | Publication date | Patent family member(s) | Publication date |
|---|---|---|---|
| EP 0507224 A2 | 07-10-1992 | AT 144701 T | 15-11-1996 |
| | | AU 654175 B2 | 27-10-1994 |
| | | CA 2064160 A1 | 28-09-1992 |
| | | DE 69214863 D1 | 05-12-1996 |
| | | DE 69214863 T2 | 10-04-1997 |
| | | DK 0507224 T3 | 07-04-1997 |
| | | ES 2095975 T3 | 01-03-1997 |
| | | GR 3022385 T3 | 30-04-1997 |
| | | JP 2536806 B2 | 25-09-1996 |
| | | JP 7010776 A | 13-01-1995 |
| EP 0355908 A1 | 28-02-1990 | NONE | |
| US 5212162 A | 18-05-1993 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002514395 T
- JP 2000239147 A
- EP 432835 A
- JP 2513506 B
- JP 2000119116 A
- US 4188373 A
- US 4478822 A
- JP 60056684 B
- JP 2873530 B
- JP 2814637 B
- JP 6067853 B

### Non-patent literature cited in the description

- *International Journal of Biological Macromolecules,* 1999, vol. 26, 255-261
- **Kimiko Abu ; Nobuko Seno.** Basics of Glycochemistry. Kodansha, 1984
- *Journal of Biologlcal Macromolecules,* 1999, vol. 26, 255-261